# EUROPÄISCHE PATENTSCHRIFT

(11) **EP 2 506 924 B1**
(45) Veröffentlichungstag und Bekanntmachung des Hinweises auf die Patenterteilung: **18.01.2017**
(21) Anmeldenummer: 10807433.7
(22) Anmeldetag: 24.11.2010
(51) Int. Cl.: A61N 1/32, A61N 1/36

(54) **VORRICHTUNG ZUR ELEKTROTHERAPEUTISCHEN BEHANDLUNG**
DEVICE FOR ELECTROTHERAPEUTIC TREATMENT
DISPOSITIF DE TRAITEMENT ÉLECTROTHÉRAPEUTIQUE

(30) Priorität: 30.11.2009 DE 102009056095
(43) Veröffentlichungstag der Anmeldung: 10.10.2012
(73) Patentinhaber: Ley, Robert, 4963 Clemency (LU); Scherer, Ralf, 66265 Heusweiler (DE)
(72) Erfinder: SCHÖNDORF, Erhard, 66787 Wadgassen (DE)
(74) Vertreter: Bernhardt, Reinhold
(86) Internationale Anmeldenummer: PCT/DE2010/001385
(87) Internationale Veröffentlichungsnummer: WO 2011/063797

(56) Entgegenhaltungen:
- EP-A1- 0 612 259
- DE-A1- 3 010 716
- FR-A1- 2 493 154
- US-A1- 2002 099 425
- US-A1- 2006 052 844
- US-A1- 2006 195 153
- A. R Ward: "Electrical Stimulation Using Kilohertz-Frequency Alternating Current", Physical Therapy, vol. 89, no. 2, 18 December 2008 (2008-12-18), pages 181-190, XP055177663, ISSN: 0031-9023, DOI: 10.2522/ptj.20080060

## Beschreibung

Die Erfindung betrifft eine Vorrichtung zur elektrotherapeutischen Behandlung des menschlichen Körpers, die an den Körper anlegbare Elektroden und eine Einrichtung zur Erzeugung eines über die Elektroden durch den Körper fließenden Therapiestroms aufweist, wobei die Einrichtung zur Therapiestromerzeugung zwei Oszillatoren mit nahe beieinander liegenden, zur Ausbildung einer Schwebung geeigneten Frequenzen f₁ und f₂, einen Oszillator mit einer Frequenz f₃, die kleiner als die Schwebungsfrequenz f_{S1} = f₁ - f₂ ist, und eine Mischereinrichtung zur Überlagerung der Oszillatorsignale umfasst.

Eine solche Vorrichtung geht aus der EP 0 612 259 B1 hervor. Zwei Wechselfelder mit Frequenzen von etwa 4000 Hz werden zu einer Schwebung mit einer Frequenz zwischen 1 und 100 Hz überlagert. Durch Überlagerung dieser Wechselfelder mit einem dritten Wechselfeld, dessen Frequenz zwischen 0,1 und 5 Hz beträgt, wird ein allmählicher Polaritätswechsel der Grundfrequenz erzeugt und Muskeln, Nerven und Gefäßgewebe mit sich sukzessive umkehrender Polung gleichstrommäßig gereizt.

Aus der DE 30 10 716 A1 ist ein Interferenzstrom-Therapiegerät bekannt, bei dem drei Wechselströme im Mittelfrequenzbereich von ca. 4 kHz überlagert werden, die sich hinsichtlich ihrer Frequenzen nur um jeweils einen geringen Betrag voneinander unterscheiden, so dass sich bei der Überlagerung Schwebungen ergeben. Die aufgrund der Überlagerung resultierenden Stromkurven haben die Form von sinusförmigen Schwebungs-Hüllkurven auf beiden Seiten der Potential-Nulllinie, wobei sich Sinushalbwellen mit großen und mit kleinen Amplituden abwechseln. Im Mittel heben sich die einander gegenüberliegenden Halbwellen der Schwebungsbäuche zu jedem Zeitpunkt potentialmäßig auf, d. h. es ergibt sich keine Gleichstromkomponente. Allerdings liegen die beiden Elektroden, die an einen zu behandelnden Muskel her angeführt werden, stets auf Potential, so dass immer ein zur Nulllinie symmetrischer Wechselstrom fließt. Der Muskel wird somit ortsmäßig stets auf die gleiche Weise behandelt. Ein Wechsel der bevorzugten Behandlungsrichtung während einer vorgegebenen Zeit findet nicht statt. Hierdurch entsteht oft eine Muskelverkrampfung, die der Patient als unangenehm empfindet.

Die US 2002/0099425 A1 beschreibt eine Vorrichtung zur Behandlung von Patienten mit elektrischen Interferenztherapie. Die Vorrichtung verwendet mehrere Elektroden zur Bereitstellung verschiedener Behandlungskanäle, wobei jeder Kanal mit einer diskreten Frequenz und eine einstellbare Schlagfrequenz zwischen allen oder ausgewählten Kanalpaaren. Die Vorrichtung ist zur endogenen Elektrostimulation entlang von zuvor festgelegter Nervenbahnen des sympathischen Nervensystems vorgesehen, wobei ein Stimulationssignal durch Überlagerung von außen eingeleiteter Grundfrequenzen im Körper erzeugt wird.

Der Erfindung liegt die Aufgabe zugrunde, bei guter Hautverträglichkeit die Wirksamkeit der elektrotherapeutischen Behandlung zu erhöhen.

Erfindungsgemäß wird diese Aufgabe dadurch gelöst, dass die Einrichtung zur Erzeugung des Therapiestroms wenigstens zwei weitere Oszillatoren mit Frequenzen f₄ und f₅ umfasst, wobei die Mischereinrichtung zur Überlagerung der Signale aller Oszillatoren mit den Frequenzen f₁ bis f₅ vorgesehen ist und die Frequenzen f₄ und f₅ zur Ausbildung einer Schwebung geeignet, wobei die Schwebungsfrequenz f_{S2} = f₄ - f₅ größer als die Schwebungsfrequenz f_{S1} ist.

Überraschend hat sich gezeigt, dass mit der erfindungsgemäßen Vorrichtung größere Ströme eingesetzt werden können, ohne dass bei einer behandelten Person an den Stellen der Haut, auf denen die Elektroden sitzen, brennende Schmerzen oder Verätzungsreaktionen auftreten. Dadurch kann die Behandlungsdauer verkürzt werden. Der Erfinder hat herausgefunden, dass durch das Zusammenwirken der Oszillatoren die Hautverträglichkeit erheblich verbessert wird und darüber hinaus auch tiefer im Körper liegende Bereiche stimuliert werden können.

Die Verwendung der beiden weiteren Oszillatoren ermöglicht es ferner, die Muskeln, die Nerven und das Gewebe in verschiedenen Frequenzbereichen zu stimulieren. Insbesondere kann mit dem Frequenzbereich von 0,1 bis 3 Hz die Lymphzirkulation angeregt werden sowie mit 1 bis 30 Hz chronische Schmerzen und bei etwa 100 Hz akute Schmerzen gelindert werden.

Ferner wird neben der Muskel- und Nervenstimulation auch das Ionenmilieu im Gewebe für eine Heilung günstig beeinflusst.

Es ist vorstellbar, durch Überlagerung mit weiteren Oszillatoren weitere Vorteile bei der Behandlung zu erzielen. Es könnten gezielt in weiteren Frequenzbereichen stimuliert werden.

In einer weiteren Ausgestaltung der Erfindung beträgt die Frequenz f_{S1} 0,1 bis 5 Hz, vorzugsweise 0,1 bis 2 Hz, und die Frequenzen f₁ und f₂ betragen zwischen 3000 und 5000 Hz, vorzugsweise zwischen 4000 und 4400 Hz. Da die Behandlung mit diesen Frequenzen auf die Schmerzrezeptoren der Haut geringe Auswirkungen hat, wird sie von den behandelten Personen nicht als unangenehm empfunden.

Zweckmäßigerweise beträgt die Frequenz f_{S2} 1 bis 100 Hz, und die Frequenzen f₄ bzw. f₅ liegen vorzugsweise ebenfalls in dem bereits genannten Bereich von 3000 bis 5000 Hz, bevorzugt 4000 bis 4400 Hz.

In einer weiteren Ausgestaltung der Erfindung ist die Frequenz f₃ ≤ (f₁ - f₂)/2. Durch den damit erzeugten, im Verhältnis zu den Schwebungen f_{S1} und f_{S2} in geringerer Frequenz stattfindenden Polaritätswechsel wird zumindest für einen gewissen Zeitraum eine gleichstrommäßige Reizung erreicht.

Zweckmäßigerweise ist die Frequenz f₃ = (f₁ -f₂)/n, wobei n ganze Zahlen ≥ 2 sind. Bei entsprechender Synchronisierung der Oszillatoren lässt sich so erreichen, dass stets vollständige Schwebungsbäuche der Schwebungsfrequenz f_{S1} durch die Überlagerungen mit dem Oszillator der Frequenz f₃ in den positiven oder negativen Bereich verschoben werden.

Besonders vorteilhafte Behandlungsergebnisse lassen sich mit der Reizstrombehandlung erreichen, wenn die Amplitude des Signals des Oszillators der Frequenz f₃ das Dreifache der sich aus der Überlagerung der anderen Oszillatorsignale ergebenden Amplitude beträgt. Dadurch wird ermöglicht, dass sich das aus der Überlagerung der Oszillatorsignale ergebende resultierende Signal für einen gewissen Zeitraum vollständig in den positiven oder negativen Bereich verschiebt.

Elektrochemische Wirkungen der Reizstrombehandlung können nunmehr besser ausgenutzt werden, wobei ein unerwünschtes dauerhaftes Verschieben von Ionen in nur eine Richtung, wie es bei der Behandlung mit Gleichstrom der Fall ist, vermieden wird. Durch diese Bewegung der Ionen kann darüber hinaus die Funktion der Osmose und Diffusion an Membranen im Körpergewebe verbessert werden.

Zweckmäßigerweise sind die Amplituden der Oszillatorsignale mit den Frequenzen f₄ und f₅ jeweils kleiner als diejenigen der Frequenzen f₁ und f₂.

In einer Ausführungsform der Erfindung ist zur Überlagerung der Oszillationssignale der Frequenzen f₁ und f₂ ein erster Mischer der Mischereinrichtung vorgesehen. Die Schwebungsfrequenz lässt sich so präzise einstellen. Ferner ist ein zweiter Mischer zur Überlagerung der vom ersten Mischer ausgegebenen Signale und der Signale der anderen Oszillatoren vorgesehen.

Zweckmäßigerweise umfasst die Einrichtung ferner einen Verstärker für die vom zweiten Mischer ausgegebenen Signale.

Die Erfindung wird nachfolgend anhand eines Ausführungsbeispiels und der beiliegenden, sich auf dieses Ausführungsbeispiel beziehenden Zeichnungen näher erläutert. Es zeigen:
- Fig. 1: ein Schaltungsschema für eine erfindungsgemäße Vorrichtung,
- Fig. 2: in einem Diagramm Signale eines ersten Mischers der Vorrichtung, und
- Fig. 3: in einem Diagramm Signale eines zweiten Mischers der Vorrichtung.

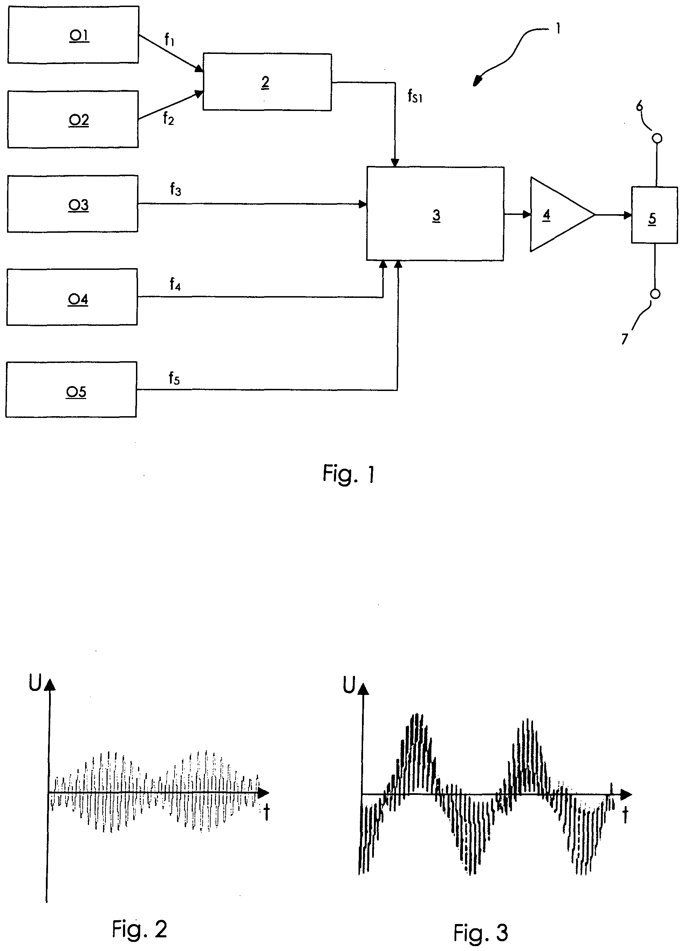

Ein Schema einer Schaltung einer Einrichtung 1 zur Strombeaufschlagung von Elektroden 6,7 einer erfindungsgemäßen Vorrichtung ist in Fig. 1 dargestellt. Es zeigt fünf Oszillatoren O₁,O₂,O₃,O₄ und O₅, zwei Mischer 2 und 3, einen Verstärker 4 sowie einen Ausgang 5 für die Elektroden 6 und 7. Die Signale der Oszillatoren O₁ und O₂, die eine Frequenz f₁ bzw. f₂ aufweisen, werden im Mischer 2 überlagert, wobei sich eine Schwebungsfrequenz f_{S1} bildet.

Die von dem Mischer 2 ausgegebene Signale sowie die Signale von O₃,O₄ und O₅ werden in dem zweiten Mischer 3 überlagert. Für ein Ausgangssignal des Mischers 3 ist ein Verstärker 4 vorgesehen, der einen Therapiestrom über den Ausgang 5 an den Elektroden 6 und 7 ausgibt.

Die Signale von O₁ bis O₅ werden normalerweise als Sinusschwingungen ausgegeben. Es sind allerdings auch andere Formen wie z.B. Dreieck- oder Rechteckschwingungen vorstellbar.

In Fig. 2 ist ein Spannungs-Zeit-Diagramm des von dem Mischer 2 ausgegebenen Signals dargestellt. Es zeigt eine Schwebung der Frequenz f_{S1} = 1 Hz und ergibt sich aus der Überlagerung der Signale von O₁ und O₂ mit einer Frequenz von f₁ = 4000 Hz bzw. f₂ = 3999 Hz. Durch entsprechendes Einstellen der Frequenzen f₁ und f₂ lässt sich die Frequenz der Schwebung zwischen 0,1 und 3 Hz ändern und je nach erforderlicher Behandlung, insbesondere zur Anregung der Lymphzirkulation, anpassen.

Die Frequenzen f₄ und f₅ der Signale O₄ und O₅ betragen 4010 Hz bzw. 4040 Hz und überlagern sich zu einer Schwebungsfrequenz f_{S2} von 30 Hz, also im Bereich der Behandlung chronischer Schmerzen. Durch entsprechende Änderung der Frequenzen f₄ und f₅ lassen sich auch hier je nach verfolgtem Behandlungsziel andere Schwebungsfrequenzen f_{S2} zwischen 1 und 100 Hz einstellen.

Wie aus Fig. 3 hervorgeht, lässt sich insbesondere durch Einstellen der Größe des Signals von O₃ auf das Dreifache der sich aus der Überlagerung der Signale von O₁,O₂,O₄ und O₅, ergebenden Spannung das insgesamt resultierende, von Mischer 3 ausgegebene Signal vollständig in den positiven oder negativen Bereich verschieben.

Die Frequenz f₃ bestimmt die Dauer, mit der eine vom Verstärker 4 ausgegebene Spannung in der jeweiligen Richtung am Körper fließt und beträgt im vorliegenden Ausführungsbeispiel 0,5 Hz und somit die Hälfte der Frequenz f_{S1}.

Zumindest für gewisse Zeiträume lässt sich somit, ohne dass an Stellen der Haut, auf denen die Elektroden sitzen, Schmerzen oder sogar Verätzungen auftreten, eine gleichstrommäßige Behandlung mit hoher Wirkung durchführen.

## Patentansprüche

1. Vorrichtung zur elektrotherapeutischen Behandlung des menschlichen Körpers, die an den Körper anlegbare Elektroden und eine Einrichtung zur Erzeugung eines über die Elektroden durch den Körper fließenden Therapiestroms aufweist, wobei die Einrichtung zur Therapiestromerzeugung zwei Oszillatoren mit nahe beieinander liegenden, zur Ausbildung einer Schwebung geeigneten Frequenzen f₁ und f₂, einen Oszillator mit einer Frequenz f₃, die kleiner als die Schwebungsfrequenz f_{S1} = f₁ - f₂ ist, und eine Mischereinrichtung zur Überlagerung der Oszillatorsignale umfasst,
**dadurch gekennzeichnet,**
**dass** die Einrichtung zur Erzeugung des Therapiestroms wenigstens zwei weitere Oszillatoren mit Frequenzen f₄ und f₅ umfasst, die Mischereinrichtung zur Überlagerung aller Oszillatoren mit den Frequenzen f₁ bis f₅ vorgesehen ist und die Frequenzen f₄ und fszur Ausbildung einer Schwebungsfrequenz f_{S2} geeignet sind, die größer als f_{S1} ist.

2. Vorrichtung nach Anspruch 1,
**dadurch gekennzeichnet,**
**dass** für ein von der Mischereinrichtung ausgegebenes Signal ein Verstärker (4) vorgesehen ist.

3. Vorrichtung nach Anspruch 1 oder 2,
**dadurch gekennzeichnet,**
**dass** die Mischereinrichtung einen ersten Mischer (2) zur Überlagerung der Oszillatorsignale mit den Frequenzen f₁ und f₂ und einen zweiten Mischer (3) zur Überlagerung eines Signals des ersten Mischers (2) mit den Oszillatorsignalen mit den Frequenzen f₃,f₄ und f₅ umfasst.

4. Vorrichtung nach einem der Ansprüche 1 bis 3,
**dadurch gekennzeichnet,**
**dass** die Mischereinrichtung zur Addition der Signale vorgesehen ist.

5. Vorrichtung nach einem der Ansprüche 1 bis 4,
**dadurch gekennzeichnet,**
**dass** die Schwebungsfrequenz f_{S1} 0,1 bis 5 Hz, vorzugsweise 0,1 bis 2 Hz, und/oder die Schwebungsfrequenz f_{S2} 1 bis 100 Hz beträgt.

6. Vorrichtung nach einem der Ansprüche 1 bis 5,
**dadurch gekennzeichnet,**
**dass** die Frequenzen f₁ und f₂ und/oder die Frequenzen f₄ und f₅ 3000 bis 5000 Hz, vorzugsweise 4000 bis 4400 Hz, betragen.

7. Vorrichtung nach einem der Ansprüche 1 bis 6,
**dadurch gekennzeichnet,**
**dass** die Frequenz f₃ ≤ (f₁ - f₂)/2 ist.

8. Vorrichtung nach einem der Ansprüche 1 bis 7,
**dadurch gekennzeichnet,**
**dass** [die Amplitude des Oszillatorsignals mit der Frequenz f₃ zumindest auf das Dreifache einer sich aus der Überlagerung der Oszillatorsignale mit den Frequenzen f₁,f₂,f₄ und f₅ bildenden Amplitude einstellbar ist.]

9. Vorrichtung nach einem der Ansprüche 1 bis 8,
**dadurch gekennzeichnet,**
**dass** die Amplitude der Oszillatorsignale mit den Frequenzen f₄ und f₅ jeweils kleiner sind als diejenigen der Frequenzen f₁ oder f₂.

## Claims

1. Apparatus for electrotherapeutic treatment of the human body, comprising electrodes which are placeable onto the body and a device for generating a therapy current which flows through the body via the electrodes, wherein the device for generating a therapy current comprises two oscillators having frequencies f₁ and f₂ which lie close together and are suitable for forming beats, an oscillator having a frequency f₃, which is lower than the beat frequency fₛ₁ = f₁ - f₂, and a mixer device for superposing the oscillator signals,
**characterized**
**in that** the device for generating the therapy current comprises at least two further oscillators having frequencies f₄ and f₅, the mixer device is provided for superposing all oscillators with the frequencies f₁ to f₅ and the frequencies f₄ and f₅ are suitable for forming a beat frequency fₛ₂, which is higher than fₛ₁.

2. Apparatus according to Claim 1,
**characterized**
**in that** an amplifier (4) is provided for a signal output by the mixer device.

3. Apparatus according to Claim 1 or 2,
**characterized**
**in that** the mixer device comprises a first mixer (2) for superposing the oscillator signals having the frequencies f₁ and f₂ and a second mixer (3) for superposing a signal from the first mixer (2) with the oscillator signals having the frequencies f₃, f₄ and f₅.

4. Apparatus according to one of Claims 1 to 3,
**characterized**
**in that** the mixer device is provided for adding the signals.

5. Apparatus according to one of Claims 1 to 4,
**characterized**
**in that** the beat frequency fₛ₁ is 0.1 to 5 Hz, preferably 0.1 to 2 Hz, and/or the beat frequency fₛ₂ is 1 to 100 Hz.

6. Apparatus according to one of Claims 1 to 5,
**characterized**
**in that** the frequencies f₁ and f₂ and/or the frequencies f₄ and f₅ are 3000 to 5000 Hz, preferably 4000 to 4400 Hz.

7. Apparatus according to one of Claims 1 to 6,
**characterized**
**in that** the frequency f₃ is ≤ (f₁-f₂)/2.

8. Apparatus according to one of Claims 1 to 7,
**characterized**
**in that** the amplitude of the oscillator signal having the frequency f₃ is adjustable to at least three times an amplitude forming from the superposition of the oscillator signals having the frequencies f₁, f₂, f₄ and f₅.

9. Apparatus according to one of Claims 1 to 8,
**characterized**
**in that** the amplitude of the oscillator signals having the frequencies f₄ and f₅ is smaller in each case than those of the frequencies f₁ or f₂.

## Revendications

1. Dispositif pour le traitement électro-thérapeutique du corps humain, qui comprend des électrodes à poser sur le corps et un système pour engendrer un courant de thérapie qui s'écoule via les électrodes à travers le corps, dans lequel le système pour engendrer un courant de thérapie inclut deux oscillateurs avec des fréquences f₁ et f₂ situées proche l'une de l'autre, appropriées pour réaliser un battement, un oscillateur avec une fréquence f₃, qui est inférieure à la fréquence de battement fₛ₁ = f₁ - f₂, et un dispositif de mélange pour superposer les signaux des oscillateurs,
**caractérisé en ce que**
le système pour engendrer le courant de thérapie inclut au moins deux autres oscillateurs avec des fréquences f₄ et f₅, le dispositif de mélange est prévu pour superposer tous les oscillateurs avec les fréquences f₁ à f₅ et les fréquences f₄ et f₅ sont appropriées pour réaliser une fréquence de battement qui est supérieure à fₛ₁.

2. Dispositif selon la revendication 1,
**caractérisé en ce qu'**il est prévu un amplificateur (4) pour un signal délivré par le dispositif de mélange.

3. Dispositif selon la revendication 1 ou 2,
**caractérisé en ce que** le dispositif de mélange inclut un premier mélangeur (2) pour superposer les signaux d'oscillateurs avec les fréquences f₁ et f₂, et un second mélangeur (3) pour superposer un signal du premier mélangeur (2) avec les signaux d'oscillateurs avec les fréquences f₃, f₄ et f₅.

4. Dispositif selon l'une des revendications 1 à 3,
**caractérisé en ce que** le dispositif de mélange est prévu pour l'addition des signaux.

5. Dispositif selon l'une des revendications 1 à 4,
**caractérisé en ce que** la fréquence de battement fₛ₁ est de 0,1 à 5 Hz, de préférence 0,1 à 2 Hz et/ou la fréquence de battement fₛ₂ est de 1 à 100 Hz.

6. Dispositif selon l'une des revendications 1 à 5,
**caractérisé en ce que** les fréquences f₁ et f₂ et/ou les fréquences f₄ et f₅ sont de 3000 à 5000 Hz, de préférence de 4000 à 4400 Hz.

7. Dispositif selon l'une des revendications 1 à 6,
**caractérisé en ce que** la fréquence f₃ est ≤ (f₁ - f₂)/2.

8. Dispositif selon l'une des revendications 1 à 7,
**caractérisé en ce que** l'amplitude du signal d'oscillateur avec la fréquence f₃ est susceptible d'être établie au moins au triple d'une amplitude formée par la superposition des signaux d'oscillateurs avec les fréquences f₁, f₂, f₄ et f₅.

9. Dispositif selon l'une des revendications 1 à 8,
**caractérisé en ce que** les amplitudes des signaux d'oscillateurs avec les fréquences f₄ et f₅ sont respectivement plus faibles que celles des fréquences f₁ ou f₂.
